# FASCICULE DE BREVET EUROPEEN

(11) **EP 2 558 583 B1**
(45) Date de publication et mention de la délivrance du brevet: **07.03.2018**
(21) Numéro de dépôt: 11719330.0
(22) Date de dépôt: 04.04.2011
(51) Int. Cl.: C12P 7/10, C12N 1/19, C12N 15/01, C12N 1/22, C12R 1/865

(54) **LEVURE INDUSTRIELLE, APTE A PRODUIRE DE L'ETHANOL A PARTIR D'AU MOINS UN PENTOSE**
INDUSTRIEHEFE ZUR HERSTELLUNG VON ETHANOL AUS MINDESTENS EINER PENTOSE
INDUSTRIAL YEAST CAPABLE OF PRODUCING ETHANOL FROM AT LEAST ONE PENTOSE

(30) Priorité: 30.04.2010 FR 1001853; 14.04.2010 FR 1001583
(43) Date de publication de la demande: 20.02.2013
(73) Titulaire: Lesaffre et Compagnie, 75001 Paris (FR)
(72) Inventeur: DESFOUGERES, Thomas, F-59960 Neuville En Ferrain (FR); PIGNEDE, Georges, F-59700 Marcq-en-Baroeul (FR); RAVE, Christophe, F-59130 Lambersart (FR); BAVOUZET, Jean-Michel, F-59170 Croix (FR); COLAVIZZA, Didier, F-59100 Roubaix (FR)
(74) Mandataire: Cabinet Plasseraud
(86) Numéro de dépôt international: PCT/FR2011/050750
(87) Numéro de publication internationale: WO 2011/128552

(56) Documents cités:
- WO-A1-2009/109634
- US-A1- 2009 246 844
- VAN MARIS A J A ET AL: "Development of Efficient Xylose Fermentation in Saccharomyces cerevisiae: Xylose Isomerase as a Key Component", ADVANCES IN BIOCHEMICAL ENGINEERING, BIOTECHNOLOGY, SPRINGER, BERLIN, DE, vol. 108, 1 janvier 2007 (2007-01-01), pages 179-204, XP008086128, ISSN: 0724-6145
- BENGTSSON O ET AL: "Xylose reductase from Pichia stipitis with altered coenzyme preference improves ethanolic xylose fermentation by recombinant Saccharomyces cerevisiae", BIOTECHNOLOGY FOR BIOFUELS, BIOMED CENTRAL LTD, LONDON UK, vol. 2, 5 mai 2009 (2009-05-05), pages 1-10, XP002612933, ISSN: 1754-6834, DOI: DOI:10.1186/1754-6834-2-9
- KUYPER MARKO ET AL: "Minimal metabolic engineering of Saccharomyces cerevisiae for efficient anaerobic xylose fermentation: a proof of principle", FEMS YEAST RESEARCH, vol. 4, no. 6, mars 2004 (2004-03), pages 655-664, XP002608429, ISSN: 1567-1356 cité dans la demande
- SONDEREGGER M ET AL: "EVOLUTIONARY ENGINEERING OF SACCHAROMYCES CEREVISIAE FOR ANAEROBIC GROWTH ON XYLOSE", APPLIED AND ENVIRONMENTAL MICROBIOLOGY, AMERICAN SOCIETY FOR MICROBIOLOGY, US LNKD- DOI:10.1128/AEM.69.4.1990-1998.2003, vol. 69, no. 4, 1 avril 2003 (2003-04-01), pages 1990-1998, XP009064914, ISSN: 0099-2240
- KUYPER M ET AL: "Evolutionary engineering of mixed-sugar utilization by a xylose-fermenting Saccharomyces cerevisiae strain", FEMS YEAST RESEARCH, WILEY-BLACKWELL PUBLISHING LTD, GB, NL LNKD- DOI:10.1016/J.FEMSYR.2005.04.004, vol. 5, no. 10, 1 juillet 2005 (2005-07-01), pages 925-934, XP004967640, ISSN: 1567-1356
- NAGAI S ET AL: "A new evidence for induction of respiration deficiency in yeast by acriflavine.", EXPERIENTIA 15 SEP 1958 LNKD- PUBMED:13586263, vol. 14, no. 9, 15 septembre 1958 (1958-09-15), pages 321-323, XP002608430, ISSN: 0014-4754
- WATANABE SEIYA ET AL: "Ethanol production from xylose by recombinant Saccharomyces cerevisiae expressing protein-engineered NADH-preferring xylose reductase from Pichia stipitis", MICROBIOLOGY (READING), vol. 153, no. Part 9, septembre 2007 (2007-09), pages 3044-3054, XP002608431, ISSN: 1350-0872 cité dans la demande
- Zorana Carter ET AL: "New generation of loxP-mutated deletion cassettes for the genetic manipulation of yeast natural isolates", Yeast, vol. 27, no. 9, 14 April 2010 (2010-04-14) , pages 765-775, XP055249050, GB ISSN: 0749-503X, DOI: 10.1002/yea.1774
- WAHLBOM C F ET AL: "GENERATION OF THE IMPROVED RECOMBINANT XYLOSE-UTILIZING SACCHAROMYCES CEREVISIAE TMB 3400 BY RANDOM MUTAGENESIS AND PHYSIOLOGICAL COMPARISON WITH PICHIA STIPITIS CBS 6054", FEMS YEAST RESEARCH, WILEY-BLACKWELL PUBLISHING LTD, GB, NL, vol. 3, 1 January 2003 (2003-01-01), pages 319-326, XP002980002, ISSN: 1567-1356, DOI: 10.1016/S1567-1356(02)00206-4

## Description

Le présent document a trait au domaine des procédés d'obtention de souches de levure productrices d'éthanol, des levures ainsi produites, et de la production industrielle d'éthanol à partir desdites levures. Plus spécialement, il concerne dans son aspect le plus général, un procédé de préparation de levures à partir de souches de *Saccharomyces cerevisiae* dites industrielles, lesdites levures et leur application à la production industrielle d'éthanol à partir de milieux industriels contenant au moins un pentose.

Le point commun des approches de l'art antérieur du domaine consiste en des procédés visant l'amélioration de souches dites de laboratoire au patrimoine génétique connu et/ou construit et dont les aptitudes à produire de l'éthanol sont étudiées en général dans des milieux et dans des conditions de laboratoire standardisées et optimales.

En effet, la littérature scientifique ainsi que les documents brevet analysés par la Demanderesse enseignent, le plus souvent, des procédés d'obtention de souches haploïdes ou diploïdes, faiblement tolérantes aux stress notamment aux fortes concentrations d'éthanol et/ou aux températures élevées et/ou à des inhibiteurs de fermentation. En outre, ces procédés nécessitent pour la plupart d'avoir recours, pour ces souches, à l'utilisation de marqueurs d'auxotrophie et/ou de marqueurs de résistance à des antibiotiques qui peuvent les disqualifier pour une utilisation ultérieure en milieu industriel pour des raisons évidentes de coût voire quelquefois de santé publique. Bengtsson et al. (Biotechnology for Biofuels, 2009, 2:9) décrit des souches de levure *Saccharomyces cerevisiae* de laboratoire présentant plusieurs modifications : surexpression des gènes de xylose réductase (XR) mutés, K270M ou K270R, et de xylitol déshydrogénase (XDH) de *Pichia stipitis*, surexpression de la xylulokinase endogène et de la voie non oxydative des pentoses phosphate, et élimination de l'activité aldose réductase par délétion du gène GRE3. Les propriétés de croissance des souches développées antérieurement sont en général insuffisantes et ces souches n'ont jamais été confrontées aux impératifs de production de biomasse à l'échelle industrielle, à savoir pour n'en citer que trois : fort taux de croissance, aptitude au séchage, stabilité en conservation.

Si des performances dites fermentaires (aptitude à la production anaérobie d'éthanol) sont obtenues dans des milieux synthétiques ou définis, dits de laboratoire, avec ces souches antérieures, elles ne sont pas transposables en général, dans des milieux industriels comportant des mélanges complexes issus par exemple de résidus de traitement de matériaux cellulosiques ou lignocellulosiques qui renferment des composés toxiques pouvant inhiber à différents niveaux la machinerie cellulaire de la levure, notamment furfural, HMF, dérivés phénoliques, acide acétique. En outre, l'aptitude au « scale up » ou transposition d'échelle de ces procédés antérieurs de production d'éthanol est rarement documentée.

La Demanderesse a ainsi constaté qu'il existe encore le besoin d'un procédé de préparation d'une levure dite industrielle qui tienne compte à la fois des contraintes du levurier et dans le même temps de celles d'un utilisateur final dans ses applications notamment en termes de production industrielle d'éthanol à faible coût et haut rendement.

C'est justement à la satisfaction de ce double besoin que vise la présente invention.

Il est décrit ici un procédé de préparation d'une souche de levure industrielle *Saccharomyces cerevisiae* apte à produire de l'éthanol à partir d'un milieu comportant au moins un pentose et qui comprend les étapes suivantes consistant à :
(i) Sélectionner et procurer une souche de levure *Saccharomyces cerevisiae* dite industrielle apte à produire de fortes concentrations d'éthanol, d'au moins 14,5% (v/v) et de préférence au moins 16% sur un hydrolysat de céréales, en conditions de Saccharification et Fermentation Simultanée (SSF) et à une température de 35°C,
(ii) Intégrer au moins une cassette d'expression ou de délétion dans le génome de la levure de l'étape (i), ladite au moins une cassette étant choisie dans le groupe constitué par :
   a. L'association du type cadre ouvert de lecture (ORF) du gène de *Pichia stipitis* XRm codant pour l'enzyme xylose réductase mutée utilisant le NADH;H+ comme co-facteur préférentiel en lieu et place du NADPH;H+ / promoteur et terminateur de *Saccharomyces cerevisiae,* ladite cassette étant flanquée en amont et en aval de régions recombinogéniques permettant son intégration ciblée dans le génome,
   b. L'association du type cadre ouvert de lecture (ORF) du gène de *Pichia stipitis* XDH codant pour l'enzyme xylitol déshydrogénase / promoteur et terminateur de *Saccharomyces cerevisiae,* ladite cassette étant flanquée en amont et en aval de régions recombinogéniques permettant son intégration ciblée dans le génome,
   c. L'association du type cadre ouvert de lecture (ORF) du gène de *Saccharomyces cerevisiae* XKS1 codant pour l'enzyme xylulokinase / promoteur et terminateur de *Saccharomyces cerevisiae,* ladite cassette étant flanquée en amont et en aval de régions recombinogéniques permettant son intégration ciblée dans le génome,
(iii) Induire l'expression d'au moins un gène de chaque étape de la partie non oxydative de la voie des pentoses phosphate en le plaçant sous la dépendance d'un promoteur d'un gène de la glycolyse fortement exprimé lors d'une fermentation alcoolique, et
(iv) Déléter au moins deux copies du cadre ouvert de lecture (ORF) du gène de *Saccharomyces cerevisiae* GRE3 codant pour une aldose déshydrogénase.

Le procédé décrit ici présente, en particulier, les avantages suivants :
Pour le fabricant de levure, il permet :
   - la construction d'une souche de levure *Saccharomyces cerevisiae* aneu/polyploïde, prototrophe afin de permettre la production de biomasse sur des sources simples de carbone, azote, phosphore dans des milieux peu chers tels que les sous-produits de l'industrie sucrière comme la mélasse par exemple,
   - de disposer d'une souche de levure *Saccharomyces cerevisiae* présentant un taux de croissance maximum (µ max) compris entre 0.37 h⁻¹ et 0.5 h⁻¹
   - de disposer d'une souche de levure *Saccharomyces cerevisiae* qui, lorsqu'elle est produite selon un procédé tel que décrit dans le livre de référence « Yeast Technology » (2ème édition, 1991, G. Reed et T.W. Nagodawithana, publié par Van Nostrand Reinhold, ISBN 0-442-31892-8), permet d'obtenir un rendement de production de biomasse d'au moins 45 g de matières sèches levure pour 100 g d'équivalent saccharose mis en oeuvre,
   - de disposer d'une souche de levure *Saccharomyces cerevisiae* résistante au procédé de séchage tel que décrit dans les documents brevets EP 511108 et US 5 741 695, la perte d'activité fermentative après séchage ne devant pas excéder 30%.
   - la production en conditions industrielles (en particulier milieu peu cher, bon rendement de biomasse, levure sèche prête à l'emploi) d'une levure fraîche ou sèche à partir d'une souche de levure *Saccharomyces cerevisiae* génétiquement stable, robuste car notamment tolérante aux concentrations élevées d'éthanol et apte à produire, à partir par exemple de biomasses hémi-cellulosiques, de l'éthanol à au moins 80 g/L et ce à une température élevée de l'ordre de 30 à 40 °C.

Par ailleurs, pour le producteur d'éthanol, l'avantage du procédé décrit ici est de disposer d'une levure active (fraîche - liquide ou comprimée - ou sèche), obtenue selon un procédé de production tel que décrit dans l'ouvrage « Yeast Technology », à partir d'une souche de levure Saccharomyces cerevisiae telle que définie au paragraphe précédent qui est :
- capable, dans les conditions de SSF décrites dans le document brevet WO 2004/046333, de fermenter à 35°C un hydrolysat de céréales jusqu'à une concentration en éthanol de 14,5 % (v/v) minimum,
- capable, dans les conditions de SSF décrites dans le document brevet WO 2004/046333, de fermenter à 35°C un hydrolysat de céréales jusqu'à une concentration en éthanol de 16 % (v/v) minimum.

Les résultats du procédé décrit ici sont d'autant plus remarquables qu'ils ont été obtenus à partir d'une souche dite industrielle, aneu/polyploïde prototrophe et de fait ayant un matériel génétique nettement plus complexe que celui d'une souche dite de laboratoire, rendant pour le moins imprévisibles les conséquences de modifications de ladite souche industrielle. Ce fond génétique complexe, spécifique aux souches industrielles, rend d'autant plus difficile l'obtention de souches génétiquement modifiées exemptes au final de marqueurs de résistance à des antibiotiques, en particulier lorsque de nombreuses cibles génétiques doivent être modifiées. Des souches exemptes de marqueurs de résistance aux antibiotiques sont, de toute évidence, préférables pour des raisons de santé et d'environnement.

La demanderesse a montré que les modifications génétiques selon le procédé décrit ici, appliquées à une souche industrielle au patrimoine génétique complexe et présentant une capacité à produire des fortes concentrations l'éthanol, n'induisent aucune instabilité génomique.

Les souches prototrophes décrites ici présentent l'avantage de croître sur des sources simples de carbone, d'azote et de phosphore.
Mais cette caractéristique fait que les vecteurs de transformation disponibles dans la communauté scientifique (vecteurs utilisant des marqueurs d'auxotrophie) sont inopérants.
Il est donc nécessaire d'avoir à disposition des outils/vecteurs utilisant des marqueurs de résistance à des antibiotiques, ces dits outils/vecteurs étant avantageusement construits pour permettre *in fine* l'excision de ces marqueurs. La construction des levures conformes à l'invention a nécessité par exemple l'emploi de 4 marqueurs positifs différents (généticine, phléomycine, hygromycine et blasticidine).

Les souches décrites ici sont aneu/polyploïdes : c'est une caractéristique généralement rencontrée dans les levures industrielles qui sont issues du milieu naturel. Le passé phylogénétique de ces souches est à l'origine de cette particularité.
Mais c'est une difficulté supplémentaire rencontrée lorsque l'on souhaite disrupter/inactiver toutes les copies d'un gène donné. Toutefois, ce caractère d'aneu-polyploïdie est en général à l'origine de beaucoup de propriétés d'intérêt des levures industrielles (taux de croissance, résistance à différents stress, stabilité phénotypique).

En outre, après de longues recherches, la demanderesse a constaté avec surprise qu'avec le procédé décrit ici, mis en oeuvre à partir de la souche dite industrielle qu'elle avait sélectionnée:
- l'introduction de cassettes d'expression et de délétion n'induisait aucune instabilité génomique chez la levure modifiée qui voit son patrimoine génétique amélioré,
- il n'était pas obligatoire de réguler son activité XK. Il existe en effet une controverse dans l'art antérieur concernant la surexpression de XKS1 dans des souches de laboratoire, donc mieux définies, qui suggère que l'activité Xylulokinase doit être finement régulée (Jin et al AEM 2003, 69,495-503 *vs* Ho et al. 1999, Advances in Biochemical Engineering/Biotechnology, Vol.65, pp. 163 - 192).

En particulier, la demanderesse a montré qu'avec ladite souche industrielle il est possible de réaliser :
- la délétion d'au moins deux copies du gène GRE3 de S. *cerevisiae* (l'enzyme Gre3p étant une aldose réductase qui consomme du NADPH;H+ qui est produit en grande partie via la partie oxydative de la voie des pentoses) dans ladite souche industrielle a permis de réduire d'autant la consommation de NADPH ;H+ par la dite enzyme,
- la surexpression de XKS1 naturellement, c'est-à-dire que l'étape c) du procédé décrit ici pourrait être omise, étant donné que XKS1 est un gène endogène de S. *cerevisiae.* Cette surexpression pourrait notamment être rendue possible après cultures cycliques lorsque le procédé comporte une étape ultérieure d'évolution dirigée, telle que décrite plus loin,

Dans certains modes de réalisation préférés, les cassettes a), b) et c) de l'étape (ii) sont toutes intégrées.

Pour ses constructions, la demanderesse a tout d'abord examiné l'effet du gène XR sauvage de *Pichia stipitis.* Après retrait des marqueurs et une étape d'évolution dirigée elle a obtenue les souches EG4 et EG5 déposée à la CNCM sous les N° CNCM I-4397 et I-4398 le 23 novembre 2010.

Cependant, même si les souches EG4 et EG5 obtenues sont plus rapides que les souches EG1 et EG2, elles produisent en moyenne 50 % de xylitol en plus. Le xylitol conduit à une diversion du carbone et réduit significativement le rendement de conversion du sucre en éthanol, ce qui est très préjudiciable compte tenu de l'application industrielle visée.

La demanderesse a ensuite remplacé le gène sauvage XR de *Pichia stipitis,* par un gène muté XRm et a constaté qu'il est préférable que le gène XRm soit un gène qui présente la mutation suivante, K270M, ou un gène XR muté qui présente une (des) mutation(s) différente(s) telle(s) que K270R décrite par Watanabe et al., Microbiol. 2007, 153, 3044-3054, de sorte que cette mutation confère à l'enzyme codée d'utiliser NADH ;H+ comme co-facteur préférentiel en lieu et place du NADPH ;H+.

La différence entre ces deux xylose réductases est que l'une porte une méthionine en position 270 (K270M) en lieu et place d'un résidu lysine alors que l'autre porte une arginine (K270R) en lieu et place d'un résidu lysine.

La demanderesse a constaté que la modification K270R réduit l'affinité de XR pour le NADPH;H+ et augmente sa capacité à utiliser le NADH;H+. De plus cette modification induit une diminution de la diversion du xylose en xylitol et permet d'améliorer le rendement de conversion du xylose en éthanol en condition de fermentation.

De manière encore plus préférée, dans cette variante, le clonage du gène XR muté (XRm), est réalisé en copie unique.

Il est également préféré dans cette variante que ledit au moins un gène de chaque étape de la partie non oxydative de la voie des pentoses phosphate de l'étape (iii) soit choisi dans le groupe constitué par *RPE1, RKI1, TKL1* et *TAL1* et que ledit promoteur d'un gène de la glycolyse fortement exprimé lors d'une fermentation alcoolique soit choisi dans le groupe constitué par le promoteur *TDH3* pour *RPE1, RKI1* et *TKL1* et *PGK1* pour *TAL1.*

Selon des caractéristiques complémentaires ou alternatives, dans le procédé de préparation d'une souche de levure industrielle *Saccharomyces cerevisiae* décrit ici :
- le promoteur à l'étape (ii) est choisi dans le groupe constitué par ADH1, ADH2, PGK1, TDH3, PDC2 et GAL1/10, de préférence *ADH1,* et le terminateur est constitué par *CYC1* ou par le terminateur propre du gène modifié, comme par exemple le terminateur TAL1 pour le gène TAL1.
- il est prévu une étape ultérieure d'évolution dirigée comportant les étapes successives suivantes consistant à soumettre la levure obtenue à
   (i) une mutagenèse,
   (ii) une croissance en cultures cycliques sous O₂ limité dans un milieu comportant ledit au moins un pentose, et
   (iii) une sélection par croissance aérobie sur milieu solide contenant comme seule source de carbone du glycérol,
de sorte à procurer des mutants non-déficients respiratoires de ladite levure qui présentent une croissance en anaérobiose en présence d'un milieu comportant ledit au moins un pentose.

De manière préférée dans cette variante, la mutagenèse de l'étape (i) est réalisée en conditions modérées, à savoir mutagenèse modérée avec 100 à 500 J/cm² et, de préférence encore, 300 J/cm² d'Ultra-violets à 254 nm. Ces conditions n'entrainent qu'une mortalité de 10 % de la population soumise aux Ultra-violets.

La demanderesse a ainsi montré de manière surprenante, qu'avec une si faible mortalité contrôlée, il est permis de réduire d'un facteur 10 la durée de l'étape d'évolution dirigée par cultures cycliques nécessaire à l'obtention de mutants capables de fermenter ledit au moins un pentose. Le taux de survie est déterminé en étalant sur boîtes gélosées contenant un milieu nutritif un volume identique de la suspension cellulaire avant et après mutagenèse. Le nombre de colonies est déterminé après 48 h de croissance.
De manière préférée, la limitation en O₂ de l'étape (ii) de cette variante est réalisée grâce à une surpression partielle dans l'équipement utilisé (par exemple, fioles ou fermenteurs) due au CO₂ produit.

Les cultures cycliques selon cette variante, dans les conditions de fermentation dudit au moins un pentose, permettent d'enrichir la population en mutants capables de fermenter ledit pentose et cela en un temps de 4 à 8 semaines et de préférence 6 semaines ce qui est relativement court et très intéressant comparé à ce qui serait obtenu par chémostat comme décrit par Kuyper *et al.* (2004) 4, 655-664.

Bien que le phénotype déficient respiratoire « petite » puisse concorder avec les critères de fermentation dudit au moins un pentose, dans cette variante la demanderesse a réalisé une étape d'élimination des levures « petites » car ce phénotype est incompatible avec les procédés de production de levures industrielles au sens de l'invention.

La présente invention a pour objet la souche de levure industrielle *Saccharomyces cerevisiae* EG3 directement obtenue par le procédé décrit ici avant l'étape d'évolution dirigée et qui consiste en la souche de levure déposée le 14 avril 2010 à la C.N.C.M (collection nationale des cultures microbiennes de l'Institut Pasteur) sous le N° I-4295 dans les conditions du traité de Budapest.
La présente invention a encore pour objet la souche de levure industrielle *Saccharomyces cerevisiae* EG2 directement obtenue par le procédé décrit ici après l'étape d'évolution dirigée et qui consiste en la souche de levure déposée le 14 avril 2010 à la C.N.C.M (collection nationale des cultures microbiennes de l'Institut Pasteur) sous le N° I-4294 dans les conditions du traité de Budapest.
La présente invention a également pour objet la souche de levure industrielle *Saccharomyces cerevisiae* EG1 directement obtenue par le procédé décrit ici après l'étape d'évolution dirigée et qui consiste en une variante incapable de sporuler de la souche de levure EG2 et déposée le 14 avril 2010 à la C.N.C.M (collection nationale des cultures microbiennes de l'Institut Pasteur) sous le N° I-4293 dans les conditions du traité de Budapest.

Une souche incapable de sporuler présente un avantage en termes de protection de l'environnement car elle supprime le risque de dissémination des transgènes par conjugaison avec d'autres levures du milieu environnant. Cette caractéristique est d'autant plus importante lorsque les microorganismes génétiquement modifiés sont utilisés à très grande échelle.
La présente invention a également pour objet la souche de levure industrielle *Saccharomyces cerevisiae* EG9 directement obtenue par le procédé décrit ici après l'étape d'évolution dirigée et qui consiste en la souche de levure déposée le 1^{er} mars 2011 à la C.N.C.M (collection nationale des cultures microbiennes de l'Institut Pasteur) sous le N° I-4450 dans les conditions du traité de Budapest.

De préférence encore,
- une souche de levure industrielle *Saccharomyces cerevisiae* obtenue est pratiquement ou totalement exempte de marqueurs notamment de résistance à des antibiotiques.

Les souches de levure *Saccharomyces cerevisiae* préparées conformément à la présente invention, selon les critères ci-dessus définis, ont conservé, après introduction des modifications génétiques et autres mutations générées lors de l'étape d'évolution dirigée, leurs caractéristiques génotypiques et phénotypiques après un procédé industriel complet de production. En particulier, les levures produites présentent une cinétique de production d'alcool, une cinétique de consommation de xylose et une quantité maximale d'alcool produite rigoureusement identiques à la souche de levure avant l'application d'un procédé industriel complet.

Par ailleurs, les caractéristiques industrielles de la souche choisie avant manipulation telles que décrites précédemment (taux de croissance, rendement de production, aptitude au séchage) demeurent inchangées.

La présente invention a encore pour objet un procédé de production d'éthanol, à partir d'un milieu comportant au moins un pentose, par fermentation à l'aide d'une levure selon l'invention, mentionnée ci-dessus. Le présent document décrit également un procédé de production d'éthanol, à partir d'un milieu comportant au moins un pentose, par fermentation à l'aide d'une levure telle qu'obtenue par un procédé comme il vient d'être décrit plus haut.
De manière préférée, le procédé de production d'éthanol présente les caractéristiques alternatives et/ou complémentaires suivantes :
- il comporte une étape de saccharification et de fermentation simultanée (SSF) en présence de polymères d'hexoses, majoritairement constitués de glucose, et d'au moins une enzyme apte à les hydrolyser,
- ledit au moins un pentose est le xylose,
- ledit milieu est choisi dans le groupe constitué par, les hydrolysats de lignine, de cellulose, d'hémicellulose, de dextrines.
- les vitesses moyennes de libération des hexoses, majoritairement du glucose, sont de l'ordre de 2,8 à 5,6 g/L/h avec une concentration extracellulaire en hexose, majoritairement en glucose, nulle.

La demanderesse a mis en oeuvre le procédé de production d'éthanol conforme à l'invention dans les conditions réelles de SSF (Fermentation et Saccharification Simultanée), telles que pratiquées dans l'industrie pour la production d'éthanol, notamment aux USA.

Les concentrations en sucres mises en oeuvre (70 g/kg de Xylose et 130 g/kg d'équivalent Glucose) sont à la connaissance de la Demanderesse, les concentrations maximum que l'on peut rencontrer sur le terrain. Tous les essais publiés faisant référence à la fermentation du xylose ont été réalisés avec des concentrations nettement inférieures en sucres totaux.

D'autres caractéristiques et avantages des souches et des procédés décrits ici apparaîtront encore mieux à la lecture de la description détaillée qui va suivre, comportant des exemples de réalisation avec tableaux de résultats qui sont donnés à titre purement illustratif et non limitatif, et pour la compréhension de laquelle on se reportera aux dessins annexés dans lesquels :
- la Figure 1 illustre un vecteur de surexpression de la XDH de *Pichia stipitis,*
- la Figure 2 est un graphe montrant le glucose libéré par hydrolyse enzymatique en fonction du temps selon trois conditions de libération initiale (A) : 2,8 g/L/h, (B) : 3,9 g/L/h et (C) : 5,6 g/L/h,
- les Figures 3 à 5 montrent pour une souche conforme à l'invention EG3, l'évolution des concentrations en glucose, xylose, éthanol, xylitol, glycérol au cours du temps, la figure 3 correspond à la dose d'enzyme A, la figure 4 à la dose d'enzyme B et la figure 5 à la dose d'enzyme C,

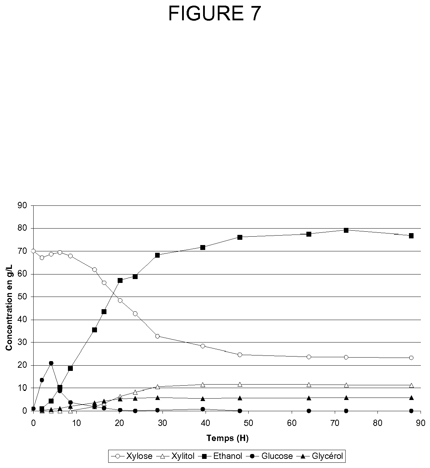

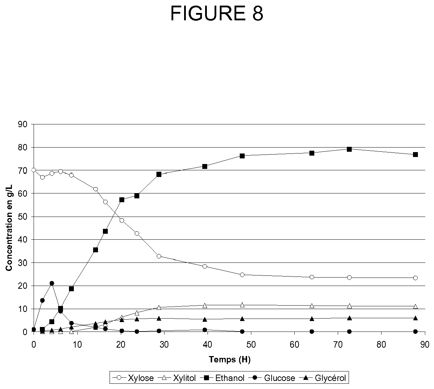

- les Figures 6 à 8 montrent pour encore une autre souche conforme à l'invention EG1, l'évolution des concentrations en glucose, xylose, éthanol, xylitol, glycérol au cours du temps, la figure 6 correspond à la dose d'enzyme A, la figure 7 à la dose d'enzyme B et la figure 8 à la dose d'enzyme C,
- la Figure 9 montre l'évolution des moyennes mobiles des vitesses de consommation du xylose par chacune des deux souches EG1 et EG3 au cours des trois essais réalisés en fonction de la moyenne mobile des concentrations en glucose dans le milieu sur le même intervalle de temps,

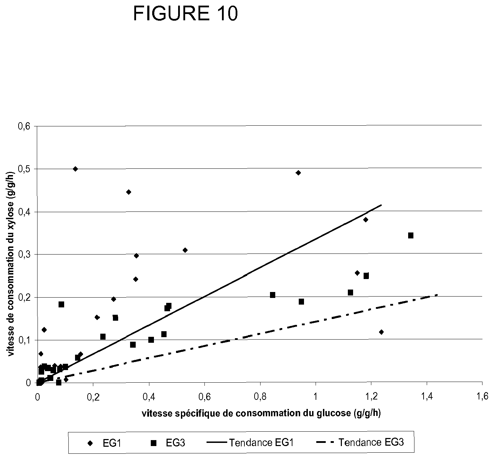

- la Figure 10 est un graphe illustrant la vitesse spécifique de production du xylitol (g/L/h) en fonction de la vitesse spécifique de consommation de xylose dans le milieu (g/L/h) pour les deux souches EG1 et EG3,

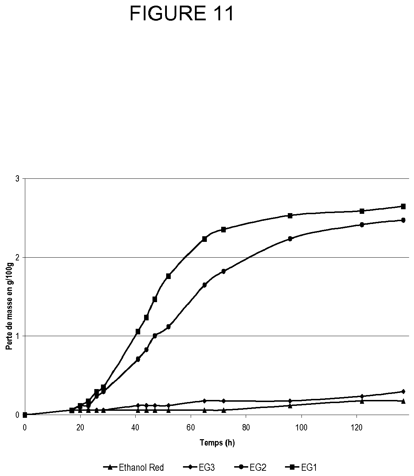

- la Figure 11 est un graphe illustrant la perte de masse en fonction du temps de fermentation en présence de xylose (70 g/L) par les 2 évoluats EG3 et EG2 (la souche Ethanol Red™ est la souche de départ).

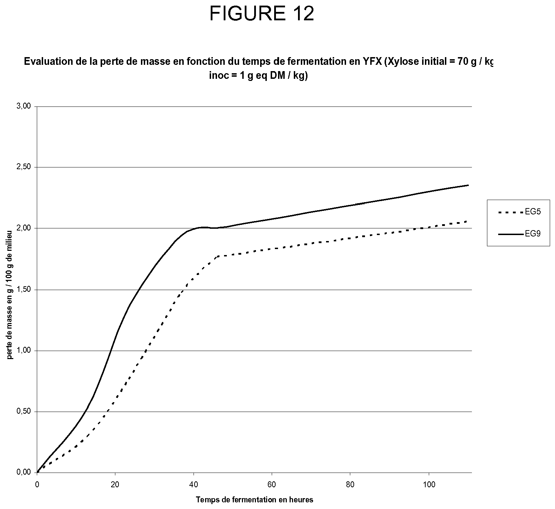

- La figure 12 illustre l'évolution de la perte de masse observée lors de la fermentation du xylose par les souches EG5 et EG9. Les cellules ont été inoculées à hauteur de 1 g / kg de matière sèche dans un milieu contenant 70 g / kg de xylose. La fermentation a été réalisée à 32 °C.

### EXEMPLES

### EXEMPLE 1

La sélection de la souche industrielle est telle que décrite dans la description ci-dessus.

Toutes les séquences d'ADN qui ont été utilisées pour les différentes transformations visant la surexpression d'un gène ont été obtenues à partir d'un vecteur-type connu (type pUC de E. coli) dans lequel ont été déclinés :
- les cibles d'intégration ;
- les promoteurs/terminateurs choisis par gène d'intérêt et
- les marqueurs de résistance qui seront éliminés par la suite (voir ci-dessous).

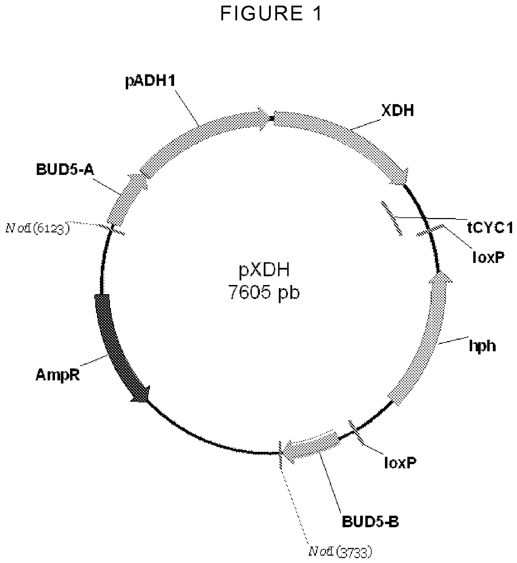

Un exemple de vecteur utilisé pour la surexpression de la XDH de *Pichia stipitis* est illustré à la Figure 1.

Pour la disruption des copies du gène GRE3 de la souche industrielle sélectionnée, la demanderesse a utilisé des amplifiats PCR à partir d'un plasmide de type pUG6 (Güldener U, Heck S, Fielder T, Beinhauer J, Hegemann JH. Nucleic Acids Res. 1996 Jul 1 ;24(13):2519-2524).

L'étape de transformation de la levure a été mise en oeuvre d'après Gietz, R.D. and R.A. Woods. (2002) Transformation of yeast by the Liac/SS Carrier DNA/PEG method. Methods in Enzymology 350: 87-96.

Les souches de levure selon l'invention, respectivement EG1, EG2 et EG3 ont été déposées à la CNCM en date du 14 avril 2010 et il leur a été attribué les Numéros I-4293, I-4294 et I-4295 respectivement.

Les souches conformes à l'invention possèdent selon un mode préférentiel le génotype suivant :
*Ethanol Red™, BUD5::ADH1p-PsXRm (K270M)-CYC1t; HO::ADH1p-PsXDH-CYC1t; BUD5::ADH1p-XKS1-CYC1t; RPE1::TDH3p-RPE1-CYC1t, RKI1::TDH3p-RKI1-CYC1t; TKL1::TDH3p-TKL1-CYC1t; TAL1::PGK1p-TAL1-CYC1t;* Δ*GRE3*

### EXEMPLE 2

La mutagenèse des souches obtenues à l'Exemple précédent a été réalisée de manière modérée à savoir de 100 à 500 J/cm² et de préférence à 300 J/cm² d'Ultra-violets à 254 nm.
Après une semaine de culture à 32 °C dans un milieu type YE (Yeast Extract 0,5 %) contenant 7 % de Xylose sous agitation, sans aération - la limitation en O₂ étant réalisée grâce à une surpression partielle dans les fioles due au CO₂ produit lors de la fermentation - un ml de la culture est utilisé pour ré-ensemencer le même milieu. Cette opération est répétée 6 fois. Les cellules sont finalement étalées sur milieu gélosé YE Glucose à 20 g/L. Des colonies isolées sont prélevées puis cultivées successivement sur :
- YE Glycérol à 20 g/L et en aérobiose pour éliminer les mutants « petite » i.e. déficients respiratoires ;
- YE Glucose pour vérifier leur vitesse de croissance ;
- YE Xylose pour identifier les clones les plus intéressants.

### EXEMPLE 3

La demanderesse a d'abord testé en culture en batch anaérobie les souches génétiquement modifiées de sorte à être capables de convertir le xylose en éthanol telles qu'obtenues à l'Exemple 2.
Elle a pu mesurer le Km apparent pour le xylose en mesurant la vitesse de production du CO₂ en fonction de la concentration en xylose et cela lors de la fermentation du xylose comme seule source de carbone : il est de 6,16 M.
Parmi les souches testées, trois sont sélectionnées en conditions SSF afin d'évaluer leur capacité à métaboliser le xylose en même temps que le glucose. Les essais SSF ont été réalisés avec des doses d'enzymes faibles dont l'activité est comprise entre 4,3 et 8,6 µKat afin que la vitesse de libération du glucose soit faible et que la concentration en glucose résiduel au cours de la fermentation soit nulle.

Les souches testées ont été la souche EG3 et la souche EG1 respectivement obtenues avant et après l'étape d'évolution dirigée.
Les cellules de la souche EG1 sont incapables de sporuler. La capacité à sporuler de ces cellules est déterminée par observation microscopique de tétrades ou d'asques obtenus en cultivant les cellules 48 h sur un milieu pauvre type SAA (Acétate de sodium 0.8%, Agar 1.5%).

### Conditions des essais.

Les essais ont été réalisés à 32 °C, pH 5. L'ensemencement a été de 0,5 g de matières sèches levure par kg de moût initial. La libération enzymatique progressive du glucose a été obtenue par utilisation de dextrines et ajout de glucoamylase. Les doses de glucoamylase utilisées ont été faibles (comprises entre 4,3 µkat et 8,6 µkat) afin de simuler une cinétique d'hydrolyse de la cellulose par des cellulases ayant lieu en 72 h. Les vitesses de libération initiale du glucose testées ont été respectivement de (A) : 2,8 g/L/h, (B) : 3,9 g/L/h et (C) : 5,6 g/L/h.

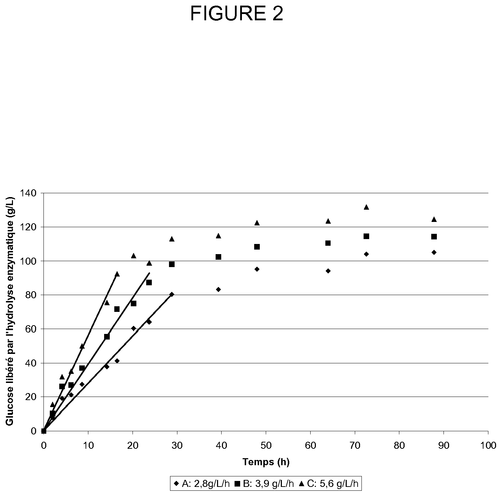

Comme observé généralement, la cinétique d'hydrolyse diminue lorsque 60-70% des dextrines ont été hydrolysées et les vitesses moyennes de libération du glucose sont ensuite de l'ordre de 0,4-0,45 g/L/h pour les trois conditions avec une vitesse légèrement plus rapide avec la condition A. (Figure 2).

En pratique, le milieu utilisé est un milieu synthétique contenant de l'extrait de levure (5 g/kg), de l'urée (2,5 g/kg), du phosphate dipotassique (1 g/kg), un tampon citrate 12 mM ainsi que des minéraux et des vitamines.

### Résultats obtenus

### Souche EG3.

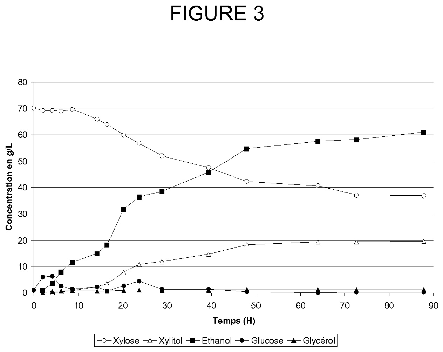

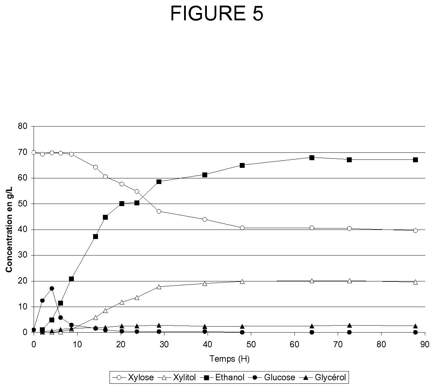

Les Figures 3 à 5 donnent l'évolution des concentrations en glucose, xylose, éthanol, xylitol, et en glycérol au cours du temps. Ces figures montrent qu'en 72 h :
- la souche EG3 a consommé entre 30 et 33 g de xylose selon les essais alors qu'elle n'en avait quasiment pas consommé en batch xylose.
- 17 à 20 g de xylitol ont été produits sur les 30-33 g de xylose consommés soit un ratio de 0,5 g/g pour la condition A et un ratio de 0,6 g/g pour les conditions B et C.
- les quantités de glycérol produit ont été faibles, plus faibles que les quantités attendues dans ce type d'essai.

Globalement, les trois essais ont donné des résultats équivalents.

**TABLEAU 1 : Molécules produites et consommées par la souche EG3 (en g par kg de milieu initial à 72 h)**

| Dose enzyme | Glucose | xylose | Biomasse | Xylitol | glycérol | Ethanol | Bilan carbone |
|---|---|---|---|---|---|---|---|
| A | 103,3 | 32,8 | 10 | 17,0 | 1,1 | 51,7 | 101% |
| B | 114,4 | 33,2 | 10 | 19,6 | 1,6 | 57,7 | 103% |
| C | 122,2 | 30,9 | 10 | 17,9 | 2,5 | 60,6 | 103% |

### Souche EG1

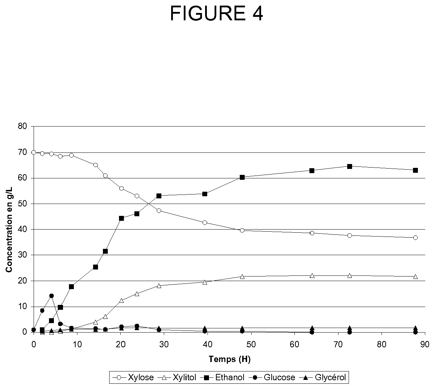

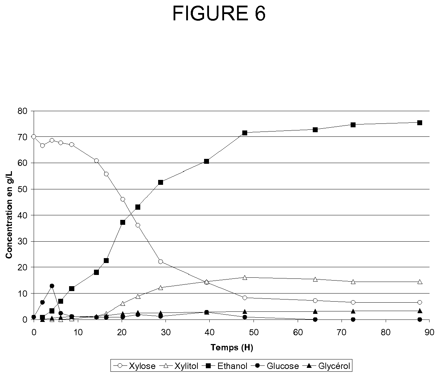

Les Figures 4 à 6 donnent l'évolution des concentrations en glucose, xylose, éthanol, xylitol, et en glycérol au cours du temps. Ces figures montrent qu'en 72 h:
- la souche EG1 a consommé entre 45 et 60 g de xylose selon les essais soit quasiment deux fois plus que la souche EG3.
- 10 à 13 g de xylitol ont été produit sur les 45-60 g de xylose consommés soit un ratio de 0,2 g/g pour les trois essais
- les quantités de glycérol produit ont été faibles mais plus élevées qu'avec la souche EG3.

Globalement, les trois essais ont donné des résultats équivalents.

**TABLEAU 2 : Molécules produites et consommées par la souche EG1 (en g par kg de milieu initial à 72 h)**

| Dose enzyme | glucose | xylose | Biomasse | Xylitol | glycérol | Ethanol | Bilan carbone |
|---|---|---|---|---|---|---|---|
| A | 102,5 | 60,61 | 10 | 12,74 | 2,86 | 65,49 | 100% |
| B | 115,6 | 51,65 | 10 | 11,90 | 4,17 | 69,34 | 102% |
| C | 120,9 | 45,96 | 10 | 9,86 | 5,33 | 70,18 | 103% |

Commentaires/hypothèses sur les résultats obtenus, notamment relatifs à la concentration en glucose permettant la prise du xylose.

Les résultats obtenus sur batch glucose-xylose montrent une rupture de pente de perte de masse qui semble indiquer que le glucose était d'abord consommé puis que le xylose était ensuite consommé à vitesse beaucoup plus lente.
L'essai SSF tel que réalisé dans l'Exemple permet d'évaluer si la prise du xylose serait plus importante en flux entrant de glucose non nul mais concentration en glucose nulle.

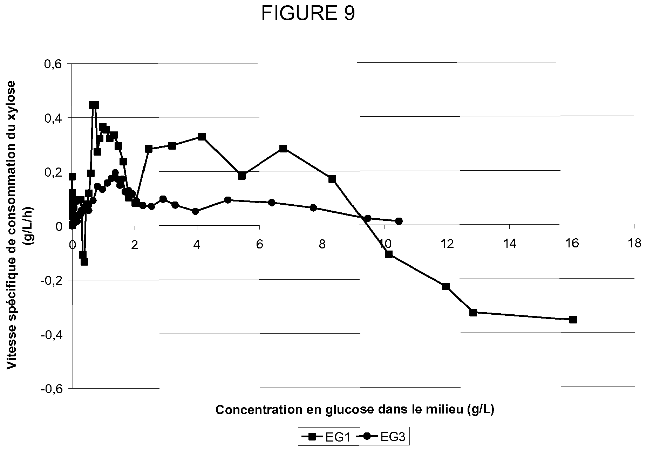

La Figure 9 montre l'évolution des moyennes mobiles des vitesses de consommation du xylose par chacune des deux souches au cours des trois essais réalisés en fonction de la moyenne mobile des concentrations en glucose dans le milieu sur le même intervalle de temps.
Les résultats permettent de constater :
- qu'il y a consommation du xylose par les souches testées vers 5 g/kg de glucose en solution,
- que la vitesse de consommation du xylose observée avec la souche EG3 est 2 fois plus faible que celles observées avec la souche EG1.

Dans une variante préférée du procédé de production d'éthanol selon l'invention, telle que présentée dans l'Exemple, la libération lente et contrôlée du glucose permet aux cellules de ne pas subir de forte variation de pression osmotique et d'éviter l'engorgement des voies fermentaires (Glycolyse, Pentoses Phosphate, et Transporteurs des sucres) qui limiterait l'utilisation du xylose.

De manière surprenante et remarquable, avec le procédé selon l'invention, les cellules sont capables de métaboliser 62 g/L de xylose en 50 heures dans un milieu très riche en sources de carbones de l'ordre de 200 g/L (Exemple réalisé avec 130 g/L d'équivalent glucose et 70 g/L de Xylose). Ces conditions aussi drastiques n'ont, à notre connaissance, jamais été décrites. D'après les essais SSF réalisés :
- la vitesse spécifique de la souche EG1 est de 0,5 g xylose/ g MS levure / h.
- 12,74 g de xylitol ont été formés pour 60,61 g de xylose consommé soit un ratio de 21 g/100 g.

## Revendications

1. Souche de levure *Saccharomyces cerevisiae* choisie parmi la souche de levure *Saccharomyces cerevisiae* EG3 déposée sous le N° I-4295 à la CNCM le 14 avril 2010, la souche de levure *Saccharomyces cerevisiae* EG2 déposée sous le N° I-4294 à la CNCM le 14 avril 2010, la souche de levure *Saccharomyces cerevisiae* EG1 déposée sous le N° I-4293 à la CNCM le 14 avril 2010, et la souche de levure *Saccharomyces cerevisiae* EG9 déposée sous le N° I-4450 à la CNCM le 1^{er} mars 2011.

2. Procédé de production d'éthanol, à partir d'un milieu comportant au moins un pentose, par fermentation d'une levure selon la revendication 1.

3. Procédé selon la revendication 2, **caractérisé en ce qu'**il comporte une étape de Saccharification et de Fermentation Simultanée (SSF) en présence de polymères d'hexoses, majoritairement constitués de glucose, et d'au moins une enzyme apte à les hydrolyser.

4. Procédé selon la revendication 2 ou la revendication 3, **caractérisé en ce que** ledit au moins un pentose est le xylose.

5. Procédé selon l'une quelconque des revendications 2 à 4 **caractérisé en ce que** ledit milieu est choisi dans le groupe constitué par les hydrolysats de lignine, de cellulose, d'hémicellulose, et de dextrines.

6. Procédé selon la revendication 5, **caractérisé en ce que** les vitesses moyennes de libération des hexoses, majoritairement du glucose, sont de l'ordre de 2,8 à 5,6 g/L/h avec une concentration extracellulaire en hexose, majoritairement en glucose, nulle.

## Patentansprüche

1. *Saccharomyces cerevisiae* Hefestamm ausgewählt aus dem *Saccharomyces cerevisiae* Hefestamm EG3 hinterlegt unter der Nummer I-4295 bei der CNCM am 14. April 2010, dem *Saccharomyces cerevisiae* Hefestamm EG2 hinterlegt unter der Nummer I-4294 bei der CNCM am 14. April 2010, dem *Saccharomyces cerevisiae* Hefestamm EG1 hinterlegt unter der Nummer I-4293 bei der CNCM am 14. April 2010 und dem *Saccharomyces cerevisiae* Hefestamm EG9 hinterlegt unter der Nummer I-4450 bei der CNCM am 1. März 2011.

2. Verfahren zur Herstellung von Ethanol aus einem Medium umfassend wenigstens eine Pentose durch Fermentation mit einer Hefe gemäß Anspruch 1.

3. Verfahren gemäß Anspruch 2, **dadurch gekennzeichnet, dass** es einen Schritt einer Simultanen Saccharifizierung und Fermentierung (SSF) in Anwesenheit von Hexosepolymeren, hauptsächlich bestehend aus Glucose, und wenigstens einem Enzym, welches diese hydrolysieren kann, umfasst.

4. Verfahren gemäß Anspruch 2 oder Anspruch 3, **dadurch gekennzeichnet, dass** die wenigstens eine Pentose Xylose ist.

5. Verfahren gemäß einem der Ansprüche 2 bis 4, **dadurch gekennzeichnet, dass** das Medium ausgewählt ist aus der Gruppe bestehend aus Hydrolysaten von Lignin, Cellulose, Hemicellulose und Dextrinen.

6. Verfahren gemäß Anspruch 5, **dadurch gekennzeichnet, dass** die mittleren Geschwindigkeiten der Freisetzung von Hexosen, hauptsächlich Glucose, in der Größenordnung von 2,8 bis 5,6 g/L/h mit einer extrazellulären Konzentration von Hexose, hauptsächlich Glucose, von Null.

## Claims

1. *Saccharomyces cerevisiae* yeast strain chosen from the *Saccharomyces cerevisiae* yeast strain EG3 deposited under the number 1-4295 at the CNCM on the 14th April 2010, the *Saccharomyces cerevisiae* yeast strain EG2 deposited under the number 1-4294 at the CNCM on the 14th April 2010, the *Saccharomyces cerevisiae* yeast strain EG1 deposited under the number 1-4293 at the CNCM on the 14th April 2010, and the *Saccharomyces cerevisiae* yeast strain EG9 deposited under the number 1-4450 at the CNCM on the 1st March 2011.

2. Process for producing ethanol from a medium comprising at least one pentose by fermentation of a yeast according to Claim 1.

3. Process according to Claim 2, **characterized in that** it comprises a step of simultaneous saccharification and fermentation (SSF) in the presence of polymers of hexoses, predominantly consisting of glucose, and of at least one enzyme capable of hydrolysing said polymers.

4. Process according to Claim 2 or Claim 3, **characterized in that** said at least one pentose is xylose.

5. Process according to any one of Claims 2 to 4, **characterized in that** said medium is selected from the group consisting of hydrolysates of lignin, of cellulose, of hemicellulose and of dextrins.

6. Process according to Claim 5, **characterized in that** the average rates of release of hexoses, predominantly of glucose, are approximately from 2.8 to 5.6 g/l/h with an extracellular concentration of hexose, predominantly of glucose, that is null.
